# EUROPEAN PATENT APPLICATION

(11) **EP 2 818 133 A1**
(43) Date of publication of application: **31.12.2014**
(21) Application number: 13173811.4
(22) Date of filing: 26.06.2013
(51) Int. Cl.: A61C 5/06, A61C 9/00

(54) **A device for dispensing dental material**

(71) Applicant: 3M Innovative Properties Company, Saint Paul, MN 55133-3427 (US)
(72) Inventor: Maurer, Andreas, 86863 Langenneufnach (DE); Pauser, Helmut, 86911 Diessen am Ammersee (DE)
(74) Representative: Hohmann, Arno

(57) **Abstract**

A device for dispensing a dental material has a cartridge and a ball-shaped closure member. The cartridge and the closure member in combination form a rotary slide valve for selectively opening and closing a cartridge outlet for the dental material, such that the device can be operated between an operative position in which the cartridge outlet is open and a storage position in which the cartridge outlet is closed. The device further has a cannula which is removably receivable within the closure member. The cannula in the operative position of the device is mechanically locked against removal. The cannula further has an annular seal which in the operative position extends into the outlet.

## Description

### Field of the Invention

The invention relates to a device for dispensing a dental material, and in particular the invention relates to a device having a rotary slide valve for selectively opening and closing the device.

### Background Art

Dental substances are often provided in packages holding a sufficient amount of substance for multiple applications. A dentist typically uses portions of such substances for application in a patient's mouth. A dentist may for example place a portion of a dental substance on a pad or into a well and use a dental instrument for applying it within a patient's mouth. Dental impression materials may for example be filled into a dental impression tray which is then placed in a patient's mouth to obtain a dental impression from the patient's teeth. However in many situations dental substances may also be placed into a patient's mouth directly from a package in which the substance is contained. Today there are a variety of packages that allow for direct application of substances into a patient's mouth.

Dental substances further are often prepared from two or more components that are mixed together just before use. Often the individual components are obtained as portions from larger packages, for example from tubes, bags or cartridges. There are packages on the market that allow manual or automatic dispensing of two or more components at desired amounts and at an appropriate ratio for mixing. Furthermore there are packages that allow dispensation of portions of readily mixed substances from individually stored components.

WO 2007/104037 discloses a dispensing device for storing and dispensing dental substances. The device comprises a cartridge for the dental substance, and a nozzle. The nozzle is pivotable with respect to the cartridge between a first position in which the capsule is closed for storage and a second position in which the capsule is opened for dispensing the dental substance.

Further WO 2010/123800 A1 discloses a dispensing device for a dental substance which has an outlet for the dental substance, and a valve for opening and closing the outlet. The device is switchable between a storage mode and an operative mode. In the storage mode a cannula is locked in the device and the valve opens the outlet, and in the storage mode the cannula can be released from the device and the valve closes the outlet.

Although there are a variety of solutions for application of dental substances into a patient's mouth there is still a desire for an application device that allows easy handling, and which is relatively inexpensive.

### Summary of the Invention

The invention relates to a device for dispensing a dental material. The device comprises:
- a cartridge and
- a ball-shaped closure member having a passageway therethrough, the passageway preferably extends along a center axis of the ball-shape of the closure member;
- the cartridge and the closure member in combination form a rotary slide valve for selectively opening and closing a cartridge outlet for the dental material;
- the rotary slide valve being operable between a storage position and an operative position, wherein in the storage position the closure member closes the cartridge outlet and in the operative position the passageway opens the cartridge outlet.

The device further comprises a cannula which comprises an application end for releasing the dental material from the device and an opposite connecting end for connecting the cannula with the cartridge outlet. The connecting end of the cannula has an annular seal.

The device is adapted for removably receiving the connecting end of the cannula within the passageway. Further in the operative position the cannula received in the passageway is mechanically locked against removal, and in the storage position removal of the cannula is enabled. In the operative position the annular seal extends into the outlet.

The device of the invention may be advantageous in that it helps keeping the dental material encapsulated even when the cannula is removed from the device. For example a user of the device may only be enabled to remove the cannula in the storage mode, in which the outlet is closed. The substances may thus be enclosed within the device. The device may thus be advantageous for storing substances over a certain storage time. The device may allow a cannula to be replaced by a fresh cannula once the device was used. Thus the device may help in fulfilling relatively strict hygiene requirements because it may allow a fresh cannula to be used for each use, for example if different patients are to be treated with the same device. Further the device may be used as a package containing multiple doses of two- or multi-component materials, for example hardenable dental materials. In that case the individual components of the substance may be co-dispensed and mixed in the cannula. Although the device is operable for opening and closing by a rotary slide valve the device preferably provides a good seal between the cannula and the cartridge due to a telescopic mating of the seal and the cartridge outlet. The ball-shape of the closure member further provides for the annular seal and the cartridge opening to form generally exact circular structures and thus to allows to maximize the sealing effect. Further the device preferably provides for a relatively convenient handling.

In one embodiment the cannula is freely insertable in the passageway and removable therefrom in the storage position. Preferably removal of the cannula is enabled only in the storage position and prohibited outside the storage position. In particular in intermediate positions between the storage and the operative position removal of the cannula is preferably blocked.

In one embodiment the cartridge and the cannula are adapted to form a bayonet lock with each other. The bayonet lock preferably provides for enabling the removal of the cannula in the storage position and prohibits removal outside the storage position. The skilled person will recognize several configurations of the cartridge and the cannula to form a bayonet lock with each other. In one embodiment the cartridge forms a keyway having a portion which is narrower than a portion (for example a retainer) of the cannula. Thus a cannula placed in the device can be retained in the device by the keyway. Further the keyway may be recessed in at least the storage position (or only in the storage position) so that the keyway in that position is larger than the same portion (or the retainer) of the cannula. Accordingly in the storage position the keyway releases the cannula so that it is freely insertable and/or removable into/from the device.

In one embodiment the cartridge forms a detent and the cannula has a retention groove so that in the operative position the detent engages the retention groove and thus locks the cannula in the device. Preferably in a so configured device in the storage position and eventually in intermediate positions between the storage position and the operative position, the detent and the retention groove are disengaged from each other.

In a further embodiment the application end of the cannula outwardly convexly tapers from a first outer diameter D1 toward a second outer diameter D2, and wherein the convex taper in a direction from D1 toward D2 tapers based on a curve that approximates a radius R which is greater than 1/2 of D1. It has been found that such a shape allows the cannula to be conveniently inserted into a sulcus in a patient's mouth. In this regard the diameter D2 may be between about 0.2 mm and about 1 mm, in particular between about 0.3 mm and about 0.7 mm, or between about 0.3 mm and about 0.8 mm, in more particular D2 may be within a range of about 0.4 mm to 0.6 mm. The diameter D2 is preferably about 0.4 mm. Further the diameter D1 may be between about 0.6 mm and about 1.5 mm, in particular between about 0.9 mm and 1.4 mm, in more particular the diameter D1 may be between about 0.9 and 1.3 mm. Preferably the diameter D1 is about 1.1 mm, or more preferably about 1.0 mm.

In one embodiment the cannula adjacent the application end has an annular marking for referencing a penetration depth of the cannula into a tissue during use of the device. Such a marking may be a printing, surface variation or a notch, for example. Thus in a treatment for dental retraction a user of the device may control the penetration depth of the cannula by help of the annular marking.

In one embodiment the annular seal protrudes from the connecting end of the cannula and tapers off toward a free end of the seal. The annular seal preferably outwardly forms a conical surface which narrows in diameter toward the free end of the seal. Further the seal preferably extends continuously annularly at a substantially triangular profile. The seal may be formed in one piece (for example monolithically) with the cannula.

In a further embodiment the cartridge, the closure member and the cannula are made of a plastic material, for example selected from one or more of polyethylene, polypropylene, acrylonitrile-butadiene-styrene terpolymer, polyamide, polybutadiene terephthalate and polyoxymethylene.

In one embodiment the cartridge forms a chamber for storing the dental material. The chamber preferably opens into an outlet channel which forms the cartridge outlet. The outlet channel may widen conically in a direction away from the chamber. Further the outlet channel may be partially formed by a lip seal of cartridge. The lip seal is preferably arranged outside of the chamber.

In a further embodiment the device comprises a piston for extruding the dental material. The piston is preferably sealingly and slidably arranged within the chamber. Further the device is adapted such that moving the piston toward the outlet causes the dental material to be extruded from the chamber in the operative position of the device.

In a further embodiment the device is adapted for storing and dispensing a two-component dental material. In this embodiment the cartridge may form two chambers for storing two components of the dental material. Further the cartridge may have two pistons for extruding the components, and the chambers may each open into an outlet channel. Each of the outlet channels preferably forms a cartridge outlet. Each of the outlet channels preferably widens conically in a direction away from the chamber. In this embodiment the cannula may have two seals protruding from the connecting end of the cannula and tapering off toward their free ends.

In one embodiment the cartridge is monolithically formed and retains the closure member by positive lock. This may be achieved by first molding the closure member and subsequently overmolding the closure member by a portion of the cartridge.

### Brief Description of the Figures

- Fig. 1: is a perspective view of a device according to an embodiment of the invention;
- Fig. 2: is an exploded view of the device shown in Fig. 1;
- Fig. 3: is a perspective view of a device according to a further embodiment of the invention;
- Fig. 4: is a cross-sectional perspective view of a device according to an embodiment of the invention;
- Fig. 5: is an enlarged cross-sectional view of details of the device shown in Fig. 4;
- Fig. 6: is a perspective view of a device according to another embodiment of the invention;
- Fig. 7: is an enlarged cross-sectional view of details of the device shown in Fig. 6; and
- Fig. 8: is a perspective view of a device according to a further embodiment of the invention.

### Detailed Description of the Invention

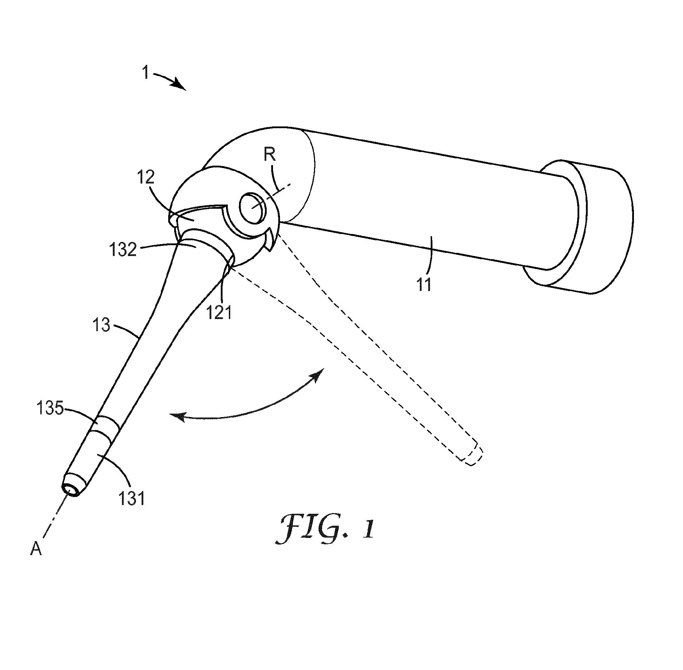

Fig. 1 shows a device 1 for dispensing a dental material. The device 1 comprises a cartridge 11 which bears a ball-shaped closure member 12. A cannula 13 is received within the closure member 12. The closure member 12 has a passageway 121 extending entirely through the closure member along a center axis A of the ball-shape of the closure member 12. The cartridge 11 and the closure member 12 in combination form a rotary slide valve. The rotary slide valve allows for selectively opening and closing the device, in particular for opening and closing a cartridge outlet (not visible in this view) for the dental material. Accordingly the rotary slide valve is operable between a storage position and an operative position. For operation between the storage position and the operative position the closure member 12 is preferably rotatable relative to the cartridge by rotation (indicated by the arrows) about a rotation axis R which is arranged generally transverse or perpendicular to the center axis A. In the storage position (indicated in dashed lines) the closure member 12 closes the cartridge outlet and in the operative position (as shown) the passageway 121 opens the cartridge outlet. The cannula 13 is received within the passageway 121 and thus extends generally along the center axis A of the closure member 12.

The cannula 13 comprises an application end 131 for releasing the dental material from the device 1 and an opposite connecting end 132 for connecting the cannula with the cartridge outlet. Further the cannula 13 has an annular marking 135 for indicating a penetration depth of the cannula 13 into tissue. This provides visibility to a user on how deep the cannula 13 penetrates into tissue, and further allows for relatively precise control of the penetration depth. This may be particularly advantageous for a dental retraction treatment in which the cannula 13 is inserted in a patient's sulcus at a predetermined penetration depth.

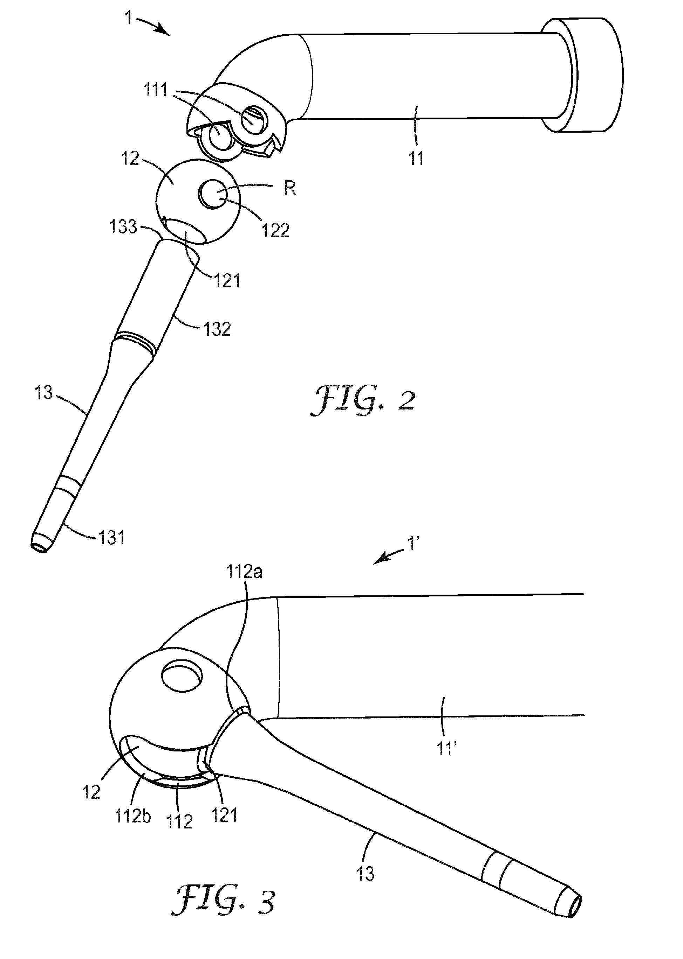

As shown in the exploded view in Fig. 2 the connecting end 132 of the cannula 13 has an annular seal 133. In particular the annular seal 133 protrudes from the connecting end 132 of the cannula 13 and tapers off toward its free end. Therefore the seal 133 is adapted such that its resilience is non-uniform over the length of the seal in a direction the seal protrudes from the connecting end 132. In particular the seal 133 thus is adapted such that the resilience of the seal 133 increases towards its free end. In the example the seal 133 further extends continuously annularly at a generally triangular profile. The free end of the seal 133 extends forms an edge of that profile. With such geometry the sealing effect of the seal may be maximized.

Further the device 1 is adapted for removably receiving the connecting end 132 of the cannula 13 within the passageway 121. Is noted that the exploded view in Fig. 2 is a virtual and schematic illustration of the components 11, 12, 13 of the device 1 separated from each other for providing a better understanding only, and that the closure member 12 and the cartridge 11 in reality are preferably not separable from each other.

The closure member 12 in the example further has two cylindrical pins 122 protruding from the closure member 12 in opposite directions about the rotation axis R. The pins 122 are adapted for retaining the closure member 12 rotatably in corresponding recesses 111 in the cartridge 11.

In the operative position the cannula 13 received in the passageway 121 is mechanically locked against removal, and in the storage position removal of the cannula 13 is enabled.

Fig. 3 shows an example in which the cannula 13 can be locked in the device 1' by a bayonet locking mechanism. The device 1' is illustrated in the storage position with a cannula 13 placed in the closure member 12. The cartridge 11' has a keyway 112 which has a first recess portion 112a which is recessed sufficiently wide to permit free insertion and/or removal of the cannula 13 in passageway 121 only in the storage position of the rotary slide valve. Further the keyway 112 has a second portion 112b forming a narrowed recess with respect to the first portion 112a. The second portion 112b extends over any position outside the storage position of the rotary slide valve. Thus the cannula 13, if inserted in the closure member 12, is locked in the device 1' at any position outside the storage position of the rotary slide valve. This enables the use of the cannula 13 for dispensing dental material from the device 1' in the operative position, and further the use of the cannula 13 to shape the dispensed material by the cannula 13 in intermediate postions between the storage and the operative position. This is because in these intermediate positions the cannula 13 is locked against removal and thus for example unintentional loss of the cannula 13 in a patient's mouth during use can be avoided.

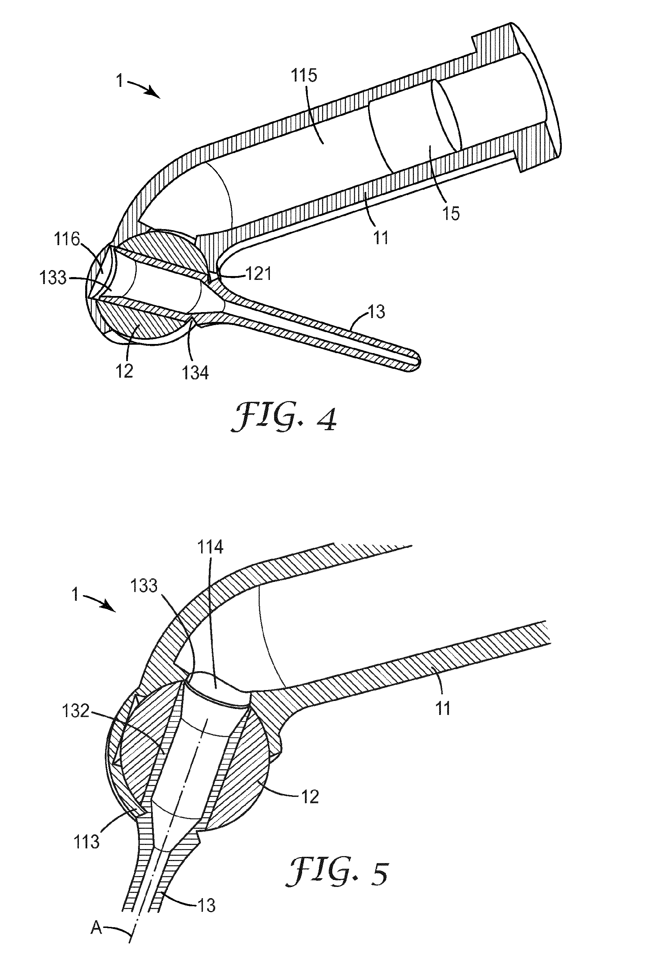

Figures 4 and 5 show the device 1 in cross-sectional views in the storage position (Fig. 4) and the operative position (Fig. 5). In the storage position shown in Fig. 4 the cannula 13 is placed in the closure member 12. In this position the cannula 13 is unlocked and thus is freely removable from the device 1. The cannula 13 has a retention groove 134 which in the storage position is disengaged from the cartridge 11. In the operative position shown in Fig. 5 a detent 113 of the cartridge 11 engages the retention groove 134 of the cannula 13 so that the cannula 13 is locked in the device 1. The embodiment in the example is adapted to lock the cannula 13 in the device 1 only in the operative position (and not in intermediate positions between the operative and the storage position), however the embodiment may be adapted to lock the cannula 13 in the device 1 in the operative position as well as in part or all of the intermediate positions between the operative position and the storage position as illustrated in Fig. 3. In the latter embodiment the detent 113 may form wall portions defining a second portion (designated as 112b in Fig. 3) of a keyway.

The length of the cannula 13 between the retention groove 134 and the free end of the annular seal 133 in the example is greater than the diameter of the ball-shaped closure member 12 along the center axis A. Further the detent 113 is shaped and arranged relative to the closure member 12 such that the retention groove 134 is engaged at a position approximately on a tangent of the outer ball surface of the closure member 12. Accordingly the seal 133 protrudes over the ball-shaped closure member 12 in the operative position of the rotary slide valve. Accordingly in the operative position the seal 133 is compressed between the detent 113 and a cartridge area at the cartridge outlet 114. Therefore a good seal may be reached between the cannula 13 and the cartridge 11. The skilled person will however recognize further arrangements which provide for the seal 133 to protrude over the ball-shaped closure member 12 in the operative position of the rotary slide valve.

The seal 133 in the example is further sized such that it fits within the cartridge outlet 114, so that in the operative position the seal 133 extends at least partly into the outlet 114. It is noted that although the seal 133 extends into the outlet 114 the seal 133 is preferably dimensioned such that it is compressed, in particular slightly compressed. Thus a good seal is established between the cannula 13 and the cartridge outlet 114 in the operative position. Dental material extruded from the cartridge 11 thus is hindered in leaking through any gap between the cannula 13 and the cartridge outlet 114. Further due to forces exerted radially on the annular seal 133 during extrusion of dental material the seal is urged laterally against the wall forming the cartridge outlet 114 the more pressure is used to extrude the dental material. Such radial forces result from pressure generated during the extrusion of the dental material and acting on the seal inwardly toward the outside. Therefore a higher extrusion force preferably does not result in a higher risk of leakage, but in the opposite a higher extrusion force is preferably automatically compensated by an increased seal effect.

As shown the device is configured such that in the storage position the seal 133 of a cannula 13 inserted in the closure member 12 protrudes into a recess 116. The cartridge 11 therefore has the recess 116 which is formed by a blind hole. The recess 116 or blind hole is arranged such that it forms a continuation of the passageway 121 in the storage position. Accordingly the recess 116 may be created during molding of the cartridge 11 over the closure member 12, with a mold core sealing and extending through the passageway 121 of the closure member 12. Thus the passageway 121 is kept free from molding material, and further the seal 133 of the cannula 13 may be prevented from being affected during insertion into the passageway 121.

The device 1 has a chamber 115 for storing a dental material. The chamber 115 is adapted for receiving a piston 15 therein, as shown in the Figure. In the example the cartridge 11 forms a generally cylindrical chamber 15 in which the piston 15 is sealingly and movably disposed. A movement of the piston 15 toward the outlet 114 therefore causes the dental material to be extruded from the chamber 115 in the operative position of the device 1. For urging the piston 15 forward (toward the outlet 114) the device can be placed into a dispensing gun (not illustrated). The device 1 therefore has an outer annular rim, allowing for the device 1 to be retained in the dispensing gun. A suitable dispensing gun is for example available under the designation Capsule Dispenser from the company 3M Deutschland GmbH, Germany.

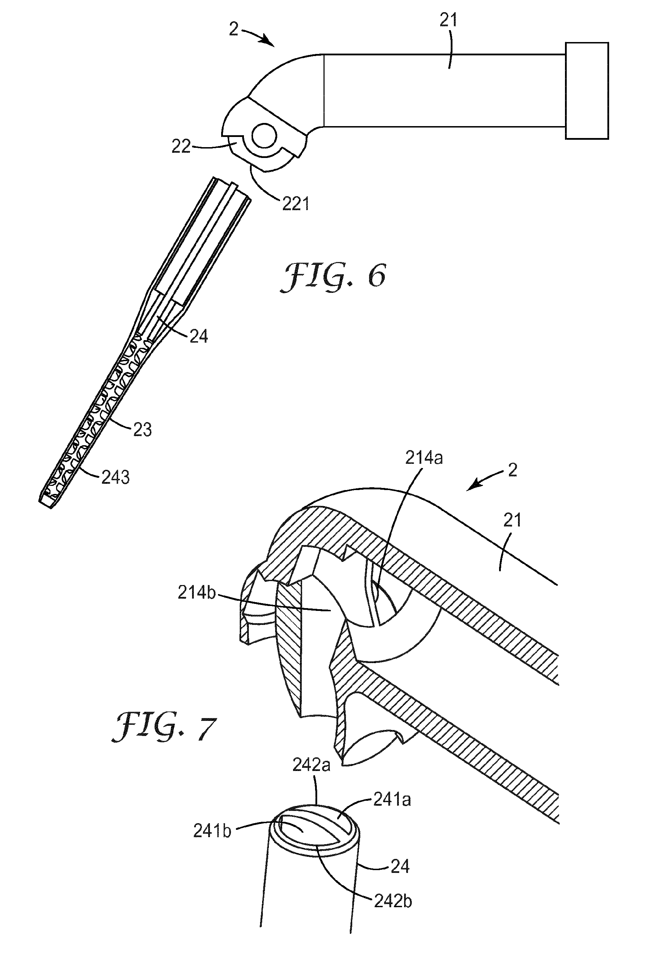

Fig. 6 shows a device 2 which generally corresponds to the device shown in Figures 1 to 5, however being adapted for dispensing and mixing a two-component dental material. The device 2 therefore has a cartridge 21 forming two chambers (not visible) for storing the two components separated from each other.

The device 21 further has a ball-shaped closure member 22, which is identical to the closure member 12 shown in Figures 1 to 5, and a cannula 23 receivable within a passageway 221 of the closure member 22. The cannula 23 in this example comprises a static mixer 24 for mixing the two-component dental material as the two components are pushed through the cannula 23.

As illustrated in Fig. 7 the device 2 further has two outlets 214a, 214b each of which forms an outlet for the component in the respective chamber (not visible in detail). Such chambers are formed in the example by the cartridge 21 and (although not illustrated) a separation wall extending inwardly along the cartridge 21. Hence the device 2 is configured such that two components can be extruded simultaneously but individually from the cartridge 21.

The mixer 24 has a first inlet 241 a and a second inlet 241b. In this example a first seal 242a and a second seal 242b are arranged at the mixer 24 around the first inlet 241 a and a second inlet 241 b. The first seal 242a and a second seal 242b are adapted to extend into and seal with the respective outlets 214a/214b in the operative position of the device 2. Accordingly the two components dispensed from the cartridge 21 flow simultaneously and individually through the respective outlets 214a/214b and the respective inlets 241 a/241 b and merge within the cannula 23. The mixer 24 has mixing blades 243 (shown in Fig. 6) which mix the two components as they flow through the cannula 23, in particular by successively merging and dividing the material resulting from the two components.

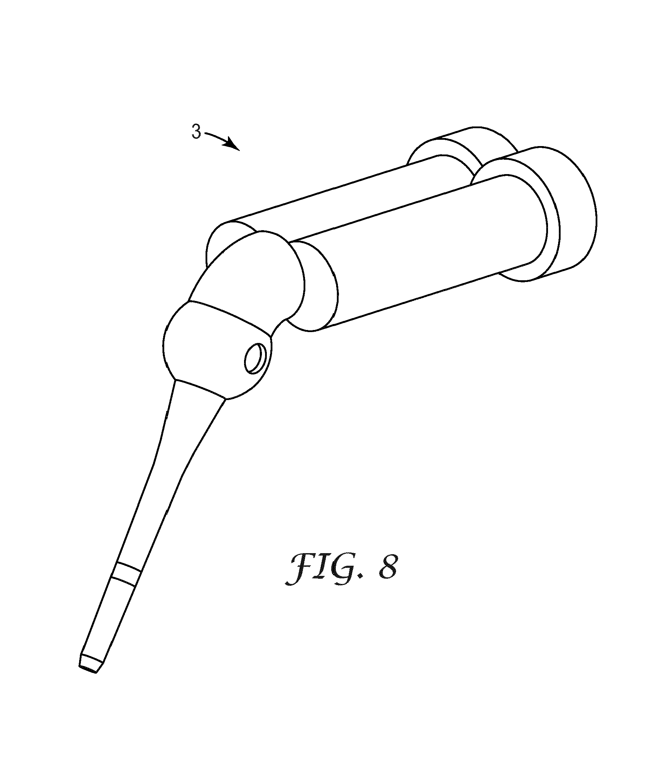

The device 2 has D-shaped chambers, outlets 214a, 214b, inlets 241 a, 241 b and seals 242a, 242b so that the overall outer profile of the device is generally circular. However in another example any of the chambers, outlets, inlets and seals may be circular. An example of a device 3 having circular chambers is illustrated in Figure 8.

## Claims

1. A device for dispensing a dental material, comprising:
- a cartridge and
- a ball-shaped closure member having a passageway therethrough,
- the cartridge and the closure member in combination forming a rotary slide valve for selectively opening and closing a cartridge outlet for the dental material;
- the rotary slide valve being operable between a storage position and an operative position, wherein in the storage position the closure member closes the cartridge outlet and in the operative position the passageway opens the cartridge outlet;
the device further comprising a cannula,
- the cannula comprising an application end for releasing the dental material from the device and an opposite connecting end for connecting the cannula with the cartridge outlet,
- the connecting end of the cannula having an annular seal;
wherein the device is adapted for removably receiving the connecting end of the cannula within the passageway;
- wherein in the operative position the cannula received in the passageway is mechanically locked against removal, and in the storage position removal of the cannula is enabled; and
- wherein in the operative position the annular seal extends into the outlet.

2. The device of claim 1, wherein the cannula is freely insertable in the passageway and removable therefrom in the storage position.

3. The device of claim 1 or 2, wherein removal of the cannula is enabled only in the storage position and prohibited outside the storage position, particularly in intermediate positions between the storage and the operative position.

4. The device of any of the preceding claims, wherein the cartridge and the cannula are adapted to form a bayonet lock with each other.

5. The device of any of the preceding claims, wherein the cartridge forms a detent and wherein the cannula has a retention groove, and wherein in the operative position the detent engages the retention groove and thus locks the cannula in the device.

6. The device of any of the preceding claims, wherein the application end of the cannula outwardly convexly tapers from a first outer diameter D1 toward a second outer diameter D2, and wherein the convex taper in a direction from D1 toward D2 tapers based on a curve that approximates a radius R which is greater than 1/2 of D1.

7. The device of any of the preceding claims, wherein the cannula adjacent the application end has an annular marking for referencing a penetration depth of the cannula into a tissue during use of the device.

8. The device of any of the preceding claims, wherein the annular seal protrudes from the connecting end of the cannula and tapers off toward a free end of the seal.

9. The device of claim 8, wherein the annular seal outwardly forms a conical surface which narrows in diameter toward the free end of the seal.

10. The device of claim 8 or 9, wherein the cartridge forms a chamber for storing the dental material, the chamber opening into an outlet channel which forms the cartridge outlet, wherein the outlet channel widens conically in a direction away from the chamber.

11. The device of any of the preceding claims, comprising a piston for extruding the dental material.

12. The device of any of the claims 1 to 7, wherein the cartridge forms two chambers for storing two components of the dental material, and two pistons for extruding the components, the chambers each opening into an outlet channel each forming a cartridge outlet, wherein each of the outlet channels widens conically in a direction away from the chamber.

13. The device of claim 12, wherein the cannula has two seals protruding from the connecting end of the cannula and tapering off toward their free ends.

14. The device of any of the preceding claims, wherein the cartridge is monolithically formed and retains the closure member by positive lock.
